# EUROPEAN PATENT SPECIFICATION

(11) **EP 3 866 758 B1**
(45) Date of publication and mention of the grant of the patent: **04.12.2024**
(21) Application number: 19798430.5
(22) Date of filing: 16.10.2019
(51) Int. Cl.: A61K 9/00, A61K 9/20

(54) **SOFTGELS WITH SOLID OR GEL-LIKE POLYMERIC FILL MATRIX**
WEICHGELE MIT FESTER ODER GELARTIGER POLYMERER FÜLLMATRIX
CAPSULES MOLLES COMPRENANT UNE MATRICE DE REMPLISSAGE POLYMÈRE SOLIDE OU DE TYPE GEL

(30) Priority: 16.10.2018 US 201862746184 P; 15.10.2019 US 201962915231 P
(43) Date of publication of application: 25.08.2021
(73) Proprietor: Bayer HealthCare LLC, Whippany, NJ 07981 (US)
(72) Inventor: ZUMETA PEREZ, Javier, 28806 Alcala De Henares (ES); PRIOR, Alberto, 28770 Colmenar Viejo (ES); CABALLO, Maria Angela, 28032 Madrid (ES); GARRIDO, Isabel Quijada, 28006 Madrid (ES); BALLESTEROS, Olga Garcia, 28006 Madrid (ES)
(74) Representative: BIP Patents
(86) International application number: PCT/US2019/056481
(87) International publication number: WO 2020/081649

(56) References cited:
- EP-A1- 2 289 493
- EP-B1- 2 833 880
- WO-A2-2004/029137
- WO-A2-2005/023181
- US-A1- 2008 243 049
- FRUTOS G ET AL: "A novel controlled drug delivery system based on pH-responsive hydrogels included in soft gelatin capsules", ACTA BIOMATERIALIA, ELSEVIER, AMSTERDAM, NL, vol. 6, no. 12, 1 December 2010 (2010-12-01), pages 4650 - 4656, XP027423389, ISSN: 1742-7061, [retrieved on 20101015], DOI: 10.1016/J.ACTBIO.2010.07.018

## Description

### FIELD OF THE INVENTION

The present invention is directed to stable dosage forms, preferably of pharmaceutical formulations, that include a solid or semi-solid polymeric mass, which entraps active ingredients and can be formulated for oral administration in a mould, preferably in a softgel capsule. The polymeric mass is obtained by polymerization of a mixture, which includes monomers, solvents, the active ingredient and polymerization initiators. A further aspect is a method for stabilizing in formulations active ingredients that are prone to hydrolysis, naproxen, acetylsalicylic acid, ibuprofen and acetaminophen or their salt forms.

### BACKGROUND OF THE INVENTION

Capsules provide dosage forms that consist of one or more active ingredients and excipients enclosed within a shell often made of gelatin. Softgels are one type of capsule, and they offer many advantages over other dosage forms. Softgel capsules are easy to swallow, can mask the taste and odor of unpleasant ingredients, present attractive appearance, are digested quickly in the gastrointestinal tract, and can be formulated in a wide variety of colors, shapes, sizes and compounds.

The "traditional" method for manufacturing a softgel product occurs in five stages: preparation of the shell mass, manufacturing of fill material, encapsulation process, drying, and finishing (see, *e.g.,* Hutchison KG, Ferdinando J. "Soft capsules". In: Aulton ME, Taylor KMG. Aulton's pharmaceutics: the design and manufacture of medicine. Elsevier Health Sciences 2013, pp 597-610).

Traditionally, the outer shell (the "shell") is prepared from gelatin, plasticizer(s) and water. Optional materials could be included into the shell formula such as opacifiers, colorants, flavors, sweeteners and preservatives. This material is shaped into the shell that forms the outermost layer of the capsule and holds the internal fill material, which typically contains the API and the excipients that are used to fill the shell itself (the "fill").

Typically, the fill within a softgel is a fluid that could be a solution, dispersion (liquid in a liquid) or a suspension (solid in a liquid). One significant problem posed by the softgels of the prior art is the use of shell material that contains water, which will degrade active ingredients and excipients that are sensitive to hydrolysis (such as aspirin or acetylsalicylic acid), due to the migration of water from the shell to the fill of the capsule. This process often leads to degradation of highly water soluble compounds and contents that are subject to hydrolysis.

In an attempt to mitigate such degradation problems, U.S. Patent No. 8,287,904 discloses a soft gelatin capsule containing aspirin, wherein a certain amount of aspirin remains in solid form suspended in a monoglyceride matrix comprising glyceryl monooleate. WO2008068276 discloses a softgel capsule with a fill that contains aspirin and another substance chosen from pharmaceutically acceptable solid polyhydroxylated organic compounds and water- soluble hygroscopic salts. And WO2017095736 combines digestible oils with surfactants in softgels containing ASA.

Another significant problem of prior art softgel capsule formulations results from interactions between compounds contained in the fill and those in the shell (see, *e.g.,* US9693978). These issues can arise from the migration of compounds between the two layers or interactions occurring at the interface between them. Such problems can begin immediately after encapsulation of the softgel fill within the shell, which occurs in an encapsulation machine.

The resulting softgels of the prior art also have excessive moisture and thus require a drying stage. Drying is a dynamic process, which continues until the gelatin shell returns to its equilibrium moisture content (10 - 15 % w/w). The entire drying process, which requires significant time, resources, and manpower, highlights another significant disadvantage of prior art softgel formulations. The softgel drying process typically occurs in two stages. First, there is a short time of low intensity drying, which combines a rotary dryer that continuously pumps dry air that is below 35 °C through a rotating drum containing the capsules. The warm air penetrates the shell moving the water outward to the softgel surface. This process can continue for approximately 1 - 3 hours depending on the formulation. The capsules are then removed from the tumble dryer to begin the secondary drying process. In the second step, the softgels are then spread onto trays, which are stacked in a drying tunnel and maintained at controlled temperature (18 - 29 °C) and low relative humidity (10 - 30 %) varying from few hours to a few days, depending on the nature of the fill formulation (see, *e.g.,* Gullapalli RP. Soft gelatin capsules (softgels). J Pharm Sci. 2010;99(10):4107-48). Traditionally, the resulting soft gelatin capsules are then sorted, polished, printed, and inspected for quality.

One potential alternative to the traditional liquid filled soft gel technology provided above is to use a polymer matrix, such as the one described by Frutos et al. (in A novel controlled drug delivery system based on pH-responsive hydrogels included in soft gelatin capsules. Acta Biomaterialia. 2010;6:4650-56). The Frutos *et al.* reference discloses a softgel fill material formed by photo-polymerization of monomers into a chemical polymer gel matrix.

Polymeric softgel matrices can be broadly categorized as physical or chemical. In the physical polymer gels, physical processes such as aggregation, crystallization, complexation or hydrogen bonding form a reversible polymer matrix. In the chemical polymer gels, chemical or covalent bonds between the monomers form the crosslinks required for polymerization and formation of the polymer matrix with permanent junctions. One significant problem of the softgel fill disclosed in Frutos *et al.* is the formation of a chemical polymer gel that behaves like an irreversible polymeric matrix in the gastrointestinal tract. The resulting fill material does not disintegrate or dissolve in the gastro-intestinal (GI) tract, which could inhibit or prevent release of active ingredients.

Another significant problem of the Frutos *et al.* fill material is the excessive swelling at physiololgical pH. The softgel fill of Frutos *et al.* contains, among other ingredients, methacrylic acid (MAA), which has a tendency to collapse at acidic pH and thus prevents the release of active ingredients in acidic environments such as the stomach and upper intestine. However, polymers obtained using MAA, have a propensity to swell in an aqueous medium that has a less acidic pH, such as that present in the intestinal environment. This property, pH sensitivity, could be used for obtaining modified-release system. Yet a further problem of the softgel fills taught in Frutos *et al.* is a significant swelling that can reach an excessive volume, which in turn can cause intestinal problems or even an intestinal obstruction. Thus, the Frutos *et al.* softgel is not suited for oral administration.

In summary, the softgel fill material of Frutos *et al.* is an irreversible chemical polymer gel which tends to collapse in the acidic environment present in the stomach and upper intestinal tract and prevents API release in those regions. It tends to swell excessively in intestinal environment and shows an inability to disintegrate or dissolve that could cause intestinal problems or blockage. Thus, the softgel capsules that contain the polymeric fill mass disclosed in Frutos *et al.* are unsuitable for human oral administration.

US 2008243049 discloses the delivery of therapeutic compounds to patient, by administering therapeutic delivery system comprising a biodegradable nanocarrier triblock copolymers or its mixtures, in combination with therapeutic compounds like naproxen or ibuprofen.

WO 2005023181 discloses a polymeric matrix as carrier for controlled delivery of an active agent like diclofenac, methacrylate derivatives used, UV-initiated polymerization is carried out using photoinitiators.

WO 2004029137 discloses polymerizable moieties such as acrylates, methacrylates, dimethacrylates. A polymerizing initiator (2,2-dimethoxy-2-phenyl acetophenone) is used and accomplished by irradiation with light at a wavelength of between about 200 to about 700 nm, diclofenac, ibuprofen and naproxen as actives used in cross-linked matrix.

EP 2289493 discloses a solid naproxen concentrate, comprising: mixing a naproxen alkali salt with at least one acidic substance (methacrylic polymers) to form a composition; filled in gelatin capsules.

EP 2833880 discloses a solid oral dosage composition comprising ibuprofen and a methacrylic acid copolymer in an amount sufficient to make the solid oral dosage composition. The matrix is formulated to be disintegrated in the oral cavity by chewing.

There is a need therefore for a softgel fill formulation that prevents or significantly reduces the migration of water from the shell to the fill mass and avoids the interaction between active ingredients. There is also a need for a softgel fill formulation that is easier to manufacture and does not require as much time and resources for drying the capsules. Further, the formulation must be capable of disintegrating or dissolving in the GI tract. In addition, there is a need for delayed release, sustained release, and immediate release formulations that have the above properties. There is also a need for using the above formulations to deliver active ingredients to a subject and to treat, for example, cough and cold, allergy, and to temporarily reduce fever, or to deliver active ingredients such as analgesics and anti-inflammatories to treat minor aches and pains due to, for example, arthritic pain, backache, headache, the common cold, muscular aches, menstrual cramps, and toothache. The present invention solves all of these problems of the prior art.

### BRIEF DESCRIPTION OF THE FIGURES

**Figure 1****.** The figure shows a comparison of dissolution testing for naproxen sodium softgel formulations made according to Frutos *et al.* versus embodiments of the present invention.
**Figure 2****.** The figure shows a comparison of swelling for naproxen sodium softgel formulations made according to Frutos *et al.* versus embodiments of the present invention.
**Figure 3****.** The figure shows dissolution testing for 3 naproxen sodium softgel formulations that are embodiments of the present invention.
**Figure 4****.** The figure shows dissolution testing for 10 additional naproxen sodium softgel formulations that are embodiments of the present invention.
**Figure 5****.** The figure shows dissolution testing for 4 naproxen acid softgel formulations that are embodiments of the present invention.
**Figure 6****.** The figure shows dissolution testing for softgel formulations containing ASA that are embodiments of the present invention.
**Figure 7****.** The figure shows dissolution testing for softgel formulations containing ASA that vary the MAA/PEG ratio and the molecular weight of PEG in the formulations.
**Figure 8****.** The figure shows dissolution testing for softgel formulations containing 500 mg of ASA.
**Figure 9****.** The figure shows dissolution testing for softgel formulations containing 600 mg of ibuprofen.

### DETAILED DESCRIPTION

The present invention solves problems of prior art by providing a softgel formulation that contains a polymeric fill matrix that is formed from activated monomers. The polymeric fill matrix of the invention does not require water; it minimizes interactions between compounds contained in the fill mass and those in the shell; it reduces water migration from the shell to the fill mass; it is sensitive to pH; it does not interact with the active ingredient; it can accommodate a wider range of excipients than those provided in the prior art; and it can disintegrate immediately or dissolve and release the active ingredient in the GI tract over time.

The present invention also provides delayed release formulations that can be used to relieve pain that arises several hours later. Some formulations, for example, can be administered prior to going to sleep for the evening while delaying release of active ingredients so that the peak activity of the API occurs prior to and during the waking hours. In this manner, the formulations can relieve or prevent discomfort or pain that arises during that time and that interferes with sleep or with transitioning from being asleep to being awake.

The present invention further includes methods for delivering active ingredients to a subject, including for example, pharmaceuticals, medicaments. It also provides methods of treating, for example, cough and cold, allergy, and to temporarily reduce fever, or to deliver active ingredients such as analgesics and anti-inflammatories to treat minor aches and pains due to, for example, arthritic pain, backache, headache, the common cold, muscular aches, menstrual cramps, and toothache. Any references in the description to methods of treatment refer to the compounds, pharmaceutical compositions and medicaments of the present invention for use in a method for treatment of the human (or animal) body by therapy (or for diagnosis).

The example methods and formulations described herein overcome one or more of the deficiencies of the prior art softgel formulations to provide a softgel fill formulation that is stable and can provide fast-release, delayed release or sustained kinetics when ingested orally.

Embodiments of the present invention utilize a polymerization technology that bypasses many of the issues associated with classical manufacturing methods and materials. For example, this invention can be extended to a broad class of active ingredients in any suitable mould, preferably in soft gelatin capsules, as demonstrated in the examples included herein.

Examples of the present invention also overcome one or more deficiencies of the prior art by providing a manufacturing method that requires considerably less time and resources to manufacture softgel formulations. The softgel manufacturing methods taught in the prior art require two drying steps, which take a significant amount of time-typically several days-depending on the nature of the fill formulation. The prior art drying steps also require significant resources, such as secondary drying steps-steps that are required to reduce capsule moisture to levels suitable for long-term storage and transport. Examples of the present invention eliminate the need for this drying step and the associated resources by providing fluid fill formulations that form and stabilize quickly. To achieve this, the present invention uses activator molecules that catalyze the conversion of polymerizable monomers into forming a "polymeric fill matrix", obtaining the final fill formulation.

In particular, in one embodiment of the invention, such formulation is based on a dosage form, preferably a pharmaceutical dosage form, that contains a mould with monomeric fill entrapping active ingredients, comprising:
a. A mould; and
b. A fluid fill mass inside the mould of (a), wherein the fill mass comprises at least one active ingredien;

at least one polymerizable monomer;
at least one polymerization initiator;
wherein the fluid fill mass does not comprise a cross linking agent andwherein activation of the polymerization initiator leads to production of a polymeric fill matrix andwherein at least one active ingredient is chosen from the group consisting of naproxen, acetylsalicylic acid, ibuprofen and acetaminophen or their salt forms andwherein the monomer is methacrylic acid and the solvent is polyethylene glycol, and wherein the ratio between monomer and solvent in the fluid fill mass is between 20:80 and 95:5.
important to note that polymerization of the monomers in the fill mass leads to a physical matrix through physical junction such as association, aggregation, crystallization, complexation or hydrogen bonding.

The resulting pharmaceutical active formulation of this embodiment of the invention also has following characteristics:
1. A wide variety of excipients compounds are compatible with the resultant polymeric matrices, unlike the limited types of excipients that are compatible with prior art softgel materials (*see*, *e.g.,* Gullapalli RP. Soft gelatin capsules (softgels). J Pharm Sci. 2010; 99(10):4107-48). The solid state of the fill material present in the polymer matrices of the present invention minimizes the migration of components between the fill mass and the outer shell, thereby reducing interactions between components contained in them. Consequently, the use of a solid or semi-solid polymeric matrix makes it possible to employ all types of gelatin shells.
2. The resulting limited interactions between the different compounds enables encapsulation of incompatible active ingredients. For example, one active ingredient can be incorporated into the shell mass, while another is incorporated into the inner fill.
3. The migration of water from the shell to fill mass during the manufacturing process and subsequent drying steps is minimized. Reducing such water migration increases the stability of hydrophilic drugs and compounds susceptible to hydrolysis, such as ASA.
4. In an embodiment of the invention, the photoinitiation step can occur at the end of the encapsulation process. The UV-Vis lamp, which catalyzes the polymerization process, can be positioned in any area of the encapsulation machine located immediately after the encapsulation step (e.g., the tumble dryer). As a result, the UV-Vis irradiation process occurs during the primary drying process, which accelerates fill mass solidification and limits compound and solvent migration. The resultant soft gelatin capsules, which are generated after the dynamic drying process, are drier (8 - 16 % weight/weight water in "dry" shell material). Such gelatin capsules do not require a secondary static drying process, thereby reducing the drying time, lowering capsule production time, eliminating the need for rooms full of drying racks and reducing manufacturing costs.

As aforementioned, one disadvantage of classical or traditional softgel capsules is stability concerns related to highly water-soluble compounds and compounds susceptible to hydrolysis, such as ASA. Such substances, which are unstable in the presence of moisture, are not stable in traditional soft gelatin capsule formulations. The solid polymeric capsules of the present invention, by contrast, provide for a solid or semi-solid fill mass that minimizes water migration from the shell to the fill mass during drying. Consequently, the stability of the active ingredients entrapped therein is significantly improved. Certain preferred embodiments of the present invention show how it is possible to achieve ASA softgel formulations that are stable and that employ a variety of ingredients and dissolution profiles.

It is further noted that, depending on the monomers chosen, the dissolution profile of the polymeric matrix can vary depending on pH-values or aqueous solubility. These properties can be varied to achieve the typical rapid release capsules, which release the encapsulated active ingredients shortly after ingestion or to obtain delayed or sustained-release capsules (see, *e.g.,* World Health Organization. Revision of monograph on capsules. Document QAS/09.339/Final. March 2011). Thus, the polymeric matrix technology of the present invention can provide a variety of formulations, with different active ingredient release profiles.

Delayed-release capsules are dosage forms that release the active ingredient into the intestinal tract and protect acid-labile drugs from the gastric environment or prevent adverse events such as irritation (*see*, *e.g.,* Stegemann S. "Capsules as delivery system for modified-release product". In: Wilson CG, Crowley PJ. Controlled Release in Oral Drug Delivery. Advances in Delivery Science and Technology 2011, pp. 277-98; Dulin W. "Oral target drug delivery systems: enteric coating". In: Wen H, Park K. Oral controlled release formulation design and drug delivery: theory to practice. John Wiley & Sons, Inc. 2010, pp. 205-23). Sustained-release capsules, on the other hand, are designed to swell in water and achieve controlled release of the active ingredient over an extended period following ingestion.

It is still further noted that in those examples that utilize photo-initiators, the photoinitiation process, which catalyzes polymerization of the monomers, does not affect the properties of the shell or mould. The properties of the mould, however, can influence the polymerization process. To ensure polymerization of the monomers present in the fill mass, the mould has to be sufficiently transparent to the wavelengths of light that activate the photoinitiators. Polymerization also depends on reactivity of monomers used, the proportion of monomers and the quantity of photoinitiator.

It is noted that in the context of the present invention, "fluid fill mass" should be understood as any acceptable solution, dispersion or suspension with an active ingredient that is suitable for filling a soft gelatin capsule and is capable of subsequently polymerizing following photoinitiation or other catalytic methods. The fluid fill mass of the present invention is free from any cross-linking agent. Said fluid fill mass usually contains at least an active ingredient, polymers, such as polyethylene glycol, polymerizable monomers and a UV-Vis photoinitiator. The active ingredient can be present in the fluid fill mass as a solute, suspension, dispersion or in equilibrium between those states.

The fluid fill mass is successfully placed in any suitable mould, preferably in a softgel capsule, or formed into the shape of a softgel prior to the final polymerization step. The softgel is preferably made of gelatin and may be any type known on the art. Soft gelatin capsules or "softgels" are, as their name implies, gelatin capsules that are softer than conventional caplets, hard capsules or tablets. They are commonly used to encapsulate liquids containing active ingredients, such as pharmaceuticals, nutrients or other consumables. Softgels are also used in many other industries, and have been used to encapsulate such diverse substances as industrial adhesives and bath oils.

The term "polymeric fill matrix" should be understood to be the polymeric softgel fill material that is formed after catalysis of the polymerizable monomers that were previously present in the fluid fill mass. The matrix is in the form of a solid or gel-like polymeric fill that entraps the active ingredients in a polymer matrix that is reversible in physiological conditions and that results from physical interactions between the polymer subunits. Such catalysis can be caused by photoinitiators, heat or other similar methods known by persons of skill in the art, as disclosed further herein.

In the present invention, the term "cross linking agent" is understood as a substance able to create chemical or covalent crosslinks between molecular chains to form a three-dimensional network of chemically or covalently connected molecules. In the absence of such a cross linking agent, physical hydrogels, which rely on physical rather than chemical interactions between the polymer subunits, are obtained. In this sense, it is important to note that the fluid fill mass inside the mould of the present invention, does not comprise a cross linking agent that would lead to a chemical hydrogel system after monomer polymerization.

It is noted that in the context of the present invention, "mould" should be understood as any suitable container of the initial monomer solution, dispersion or suspension. Please note that "moulds" in this context not only encompasses pharmaceutically acceptable ingredients such as soft gelatin capsules, but also any other inert compositions that are suitable in the pharmaceutical industry, such as a blisters, and that are capable of providing for a suitable container of the initial monomer mixture. They also include the type of seamless capsule shells formed using the droplet method described in U.S. Patent No. 7,226,613. Common "inert" materials and containers that can be used as moulds for polymerization include those made of plastic or glass; metallic materials could also be used. The only requirement for those embodiments where the polymeric fill matrix forms through photo-polymerization is that the mould material should be sufficiently transparent to the light wavelength required to catalyze photo-polymerization that polymerization of the monomers is achieved. Typical materials that can be used as containers for polymerization are: aclar, PVC, PVDC, PP, PET and COC. Metallic materials as aluminum could also be used.

In the context of the present invention, "chemical bond" refers to a covalent link between two or more molecules through a chemical reaction, such as those present in the Frutos *et al.* formulation. Such chemical bonds are irreversible in the mammalian gastrointestinal tract.

In the present invention, "physical bonds" or "physical interactions between polymer subunits" refers to physical interactions between molecules, such as those formed through association, aggregation, crystallization, complexation or hydrogen bonding. Such bonds or interactions are reversible in the gastrointestinal tract.

In the present invention, "reversible polymer" refers to a polymer that can dissolve or disintigrate in the GI tract.

In the present invention, "active ingredients" refers to any component of a drug product intended to furnish pharmacological activity or other direct effect in the diagnosis, cure, mitigation, treatment, or prevention of disease, or to affect the structure or any function of the body of humans or other animals. Active ingredients include those components of the product that may undergo chemical change during the manufacture of the drug product and be present in the drug product in a modified form intended to furnish the specified activity or effect and may include for example biologically active substances such as pharmaceuticals, medicaments, minerals, nutraceuticals, vitamins, supplements, amino acids, antioxidants, and similar materials.

### Fill Materials

It is thus noted that the fluid fill mass of the invention is a solution, dispersion or suspension that contains polymerizable monomer(s), active ingredients, solvents, polymerization initiators, wherein the fluid fill mass does not comprise a cross linking agent, and, optionally, other additive(s) like co-solvents.

### Polymerizable Monomers

Polymerizable monomers include, but are not limited to, one or more (meth)acrylic and vinylic monomers, typically: methacrylic acid (MAA), acrylic acid, methyl methacrylate, hydroxyethyl (meth)acylate, hydroxypropyl (meth)acrylate, polyethylene glycol (meth)acrylates, polypropylene glycol (meth)acrylates monomers with ethylene glycol lateral chains, styrene, vinylpirrolidone, PPG methacrylate (PPGMA) or vinyl alcohol. In a particularly preferred embodiment, the fluid fill mass inside the softgel shell comprises a single species of polymerizable monomer. In particular, such single species is selected from the list consisting of (meth)acrylic and vinylic monomers, typically: methacrylic acid, acrylic acid, methyl methacrylate, hydroxyethyl (meth)acrylate, hydroxypropyl (meth)acrylate, polyethylene glycol (meth)acrylates, polypropylene glycol (meth)acrylates monomers with ethylene glycol lateral chains, styrene, vinylpyrrolidone or vinyl alcohol. The polymerizable monomers of the invention are methacrylic acid (MAA), which can serve to obtain gastro-resistant polymeric matrices.

The polymerizable monomer of the present invention preferably comprises from about 10% by weight to about 99% by weight of the fluid fill mass. More preferably, the monomeric material comprises from about 15% by weight to about 99% by weight of the fluid fill mass, and even more preferably, the monomeric material comprises from about 20% by weight to about 99% by weight of the fluid fill mass. Most preferably, the monomeric material comprises from about 30% by weight to about 99% by weight of the fluid fill mass. In a preferred embodiment, the polymerizable monomer comprises from about 20% by weight to about 70% by weight of the fluid fill mass. In a particularly preferred embodiment, the polymerizable monomer comprises from about 20% by weight to about 60% by weight of the fluid fill mass. In a particularly preferred embodiment, the polymerizable monomer comprises from about 20% by weight to about 30% by weight of the fluid fill mass. In a particularly preferred embodiment, the polymerizable monomer comprises from about 50% by weight to about 65% by weight of the fluid fill mass. In a particularly preferred embodiment, the polymerizable monomer comprises from about 15% by weight to about 25% by weight of the fluid fill mass. When blends of monomer species are used, it is preferable, but not critical, that one species predominates.

The inventors have discovered that in certain preferred embodiments the types of monomers, the monomer to solvent ratios, and the ratio of monomers to salt forms thereof is important to the performance of the claimed invention:
- Elimination of certain polymerizable monomers used in the prior art. The closest prior art fomulations combined MAA and polyethylene glycol methyl ether methacrylate (̵PEGMEMA) to form a polymeric hydrogel, but the resulting formulations exhibited swelling behavior in the gastro intestinal environment that makes them unsuitable for oral drug administration. The inventors discovered that eliminating PEGMEMA from the formulation and using different monomers, methacrylic acid (MAA), generated polymeric hydrogels that are well-suited for use in oral softgel formulations.
- Identification of optimal monomer and solvent ratios. In certain embodiments, variations in the MAA and solvent ratios incorporated into the fluid fill mass can also affect the swelling/dissolution of polymeric fill matrix. For example, the inventors discovered that certain formulations with a high PEG concentration combined with MAA as a monomer have a faster dissolution profile and are thus well-suited for use in immediate release formulations.
- Substitution of the polymerizable monomer with its salt form. The inventors also discovered that substituting some MAA with its sodium salt form (sodium methacrylate, MAA⁻ Na⁺) can reduce the degree of physical crosslinking and thus favor polymer matrix disintegration/dissolution in some embodiments. This too can serve to facilitate active ingredient release in certain formulations.

The inventors have discovered that replacing PEGMEMA in certain prior art softgel formulations (particularly those that combined MAA with PEGMEMA as polymerizable monomers) facilitates disintegration or dissolution of the polymeric fill matrix in physiological conditions. In particular, in embodiments containing PEG instead of PEGMEMA, the MAA carboxylic group is protonated at acidic pH, forming hydrogen bridges with other acid groups (collapsed state), which prevents disintegration or dissolution of the filler in this medium. However, at pH 6.8 the MAA carboxylic group is in its ionized form, with a repulsive force between charges. This allows the medium to enter, increasing osmotic pressure in the polymer matrix and favoring its disintegration or dissolution. Furthermore, the entry of water in the system favors the dissolution of PEG. Capsules manifesting this embodiment of the present invention dissolved after 3 hours at pH 6.8 (4 hours after being subjected to an acidic medium for 2 hours) [See Real Farmacopea Española 2008. Monograph 2.9.1].

### Solvents

The invention comprises a solvent, preferably one that comprises a low molecular weight polymeric material. As used herein, "a low molecular weight" polymer is any polymer that is liquid or semi-solid at about room temperature and pressure or any polymer that can dissolve in a limited amount of water to solubilize the other ingredients. The particular identity of the polymeric material selected as a solvent will guide one skilled in the art to the appropriate molecular weight for the polymer. Since the polymer will be ingested, it must be safe and nontoxic (at least when used in the amounts contemplated herein). In addition, while such polymers need not be organoleptically pleasing, they preferably do not cause detrimental effects following ingestion. Such solvents can dissolve or suspend the active ingredients of the present invention alone or in combination with other ingredients as part of a "solvent system." The solvent for use in the present invention is polyethylene glycol.

It is understood by a person of skill that linear or branched polymers used as solvents in the present invention generally do not have a single molecular weight. Rather, each strand in a polymer sample will have a different length, and the "molecular weight" of a polymer sample will be the average molecular weight of the strands.

Acceptable polymers used in the invention include: polyethylene glycols. The polyethylene glycols of the invention have a molecular weight of less than about 1,500, since polyethylene glycol 1,500 is reported to be solid at room temperature. Molecular weights of about 1,500 or above are not excluded from the invention to the extent that the polymer may be liquid or soluble in limited amounts of water. Most preferably, the molecular weight of the polyethylene glycol is from about 400 to about 600 daltons, and the most preferred embodiment of the invention uses polyethylene glycol having a molecular weight of about 400 or 600. The solvent may comprise mixtures of materials as well. For example, a polyethylene glycol having a molecular weight of about 600 may be obtained by using PEG 600 or about a 50/50 mixture of PEG 400 and PEG 800.

Additional acceptable excipients suitable for use in the solvent system of the claimed invention include, but are not limited to, ingestible oils, vegetable oils, and alcohols, such as ethanol, propanol and higher alcohols. Disintegrants or low molecular weight water-soluble polymers can also be incorporated into the present invention to reduce disintegration or dissolution time or to improve drug solubility. The excipients that may be used in certain embodiments of the present invention depend on the particular active ingredients. For example, water is limited or excluded entirely from those embodiments of the present invention that contain active ingredients, like ASA, that degrade in the presence of water. In a further embodiment, the polymeric system of the invention may also contain additional ingredients such as co-solvents, including, as already stated, dimethyl isosorbide and oils, including soybean oil. The co-solvent may comprise from 0% by weight to about 30% by weight of the fluid fill mass of the invention, and more preferably from about 5% by weight to about 20% by weight of the fluid fill mass.

Such solvent systems preferably comprise from about 0% by weight to about 89% by weight of the fluid fill mass of the invention. More preferably, the solvent system comprises from about 15% by weight to about 65% by weight of the fluid fill mass and even more preferably, the solvent system comprises from about 20% by weight to about 60% by weight of the fluid fill mass. Most preferably, the solvent system comprises from about 25% by weight to about 60% by weight of the fluid fill mass. In a preferred embodiment, the solvent system comprises from about 5% by weight to about 10% by weight of the fluid fill mass. In another preferred embodiment, the solvent system comprises from about 40% by weight to about 50% by weight of the fluid fill mass. In another preferred embodiment, the solvent system comprises from about 30% by weight to about 40% by weight of the fluid fill mass. When polymer blends are used in the solvent system, it is preferable, but not critical, that one species of polymer predominates. Thus, in one preferred embodiment of the invention, the solvent system comprises from about 15% to about 65% by weight polyethylene glycol 600 and from 0% to about 5% by weight of (and more preferably from 0% to about 2% by weight) propylene glycol.

### Active Ingredients

The active ingredient(s) of the invention are intended to furnish pharmacological activity or other direct effect in the diagnosis, cure, mitigation, treatment, or prevention of disease, or to affect the structure or any function of the body of humans or other animals. At least one active ingredient is chosen from aspirin, acetaminophen, naproxen, and ibuprofen).

The total amount of active ingredients of the invention may comprise from about 1% by weight of the fluid fill mass up to the amount that will form a fully saturated solution, usually up to about 70% by weight of the fluid fill mass. Preferably, however, the active ingredients comprise from about 10% by weight to about 50% by weight of the fluid fill mass of the invention.

The active ingredient should remain in solution, dispersion or suspension to achieve the benefits of the invention, and the solution should remain stable until the polymerization process. The fluid fill mass disclosed in the present invention has been found to be stable and robust in a number of tests until polymerization, as illustrated in the examples.

### Polymerization Initiators

In a preferred embodiment of the invention, polymerization is catalyzed, often by a photoinitiator. In some embodiments, the photoinitiator is a substance that creates reactive species that initiate polymerization of the monomers present in the fluid fill mass when exposed to radiation in the visible light to UV spectrum. Optimal photoinitiators will be those showing an absorption wavelength similar to the selected bulb emission wavelength. Such photoinitiators include, for example, 2,2-dimethoxy-2-phenylacetophenone (DMPA; trade name irgacure 651), α-hydroxy-ketones, benzophenone derivatives, di-methylbenzyl phenone, 2-hydroxy-2-methyl-propiophenone (trade name darocur 1173), 1-hydroxycyclohexyl-phenyl ketone (trade name Irgacure 184), 1-[4-(2-Hydroxyethoxy)-phenyl]-2-hydroxy-2-methyl-1-propane-1-one (trade name Irgacure 2959) and 1-hydroxy-cyclohexyl-phenyl-ketone/benzophenone 50/50 wt % (trade name irgacure 500). Irgacure 651 is one particularly preferred photoinitiator, but any other photoinitiator capable of absorbing UV light or even visible light (camphorquinone derivatives) could also be used. The inventors have found DMPA to be a particularly useful photoinitiator for certain embodiments of the claimed invention due to its high absorption of radiation at wavelengths closest to 365 nm.

In other embodiments of the present invention, polymerization can be initiated using thermal initiator(s) (such as azobisisobutyronitrile) or redox initiator(s) (such as benzoyl peroxide), both of which are well known in the art. The initiators can be used alone or in combination with other initiators such as photoinitiators.

In the preferred embodiments that use photoinitiators, the fluid fill mass should be irradiated at a specific ultraviolet or visible range of wavelength to generate reactive species to initiate polymerization, turn monomers into a polymer and change the fluid fill mass into the polymeric fill matrix of the final formulation. The irradiation process should not affect the properties of any mould present in that particular embodiment and any such mould should be sufficiently transparent to allow the penetration of the correct wavelength of light. In preferred embodiments, the photoinitiator may comprise between 0% by weight and 5% by weight of the fluid fill mass of the invention, and more preferably from about 0.1% by weight to about 2% by weight of the fluid fill mass. In another preferred embodiment, the photoinitiator may comprise between 0.1% by weight and 1 % by weight of the fluid fill mass of the invention.

It is noted that the selection of ingredients to be used in the system of the present invention will, of course, depend on the active ingredient to be administered. Different active ingredients, such as naproxen, aspirin and ibuprofen, have different chemical structures and different affinities for various solvent combinations. Highly concentrated solutions and suspensions of active ingredients, such as aspirin and naproxen, require a solvent system tailored to the specific needs of the active. This is illustrated in the examples of the present invention.

### Other optional excipients

Additional excipients that may be useful for the matrix fills described herein include, for example, the following: acidifying agents (such as lactic acid, phosphoric acid, acetic acid, citric acid, fumaric acid, hydrochloric acid, malic acid, nitric acid, sulfuric acid, tartaric acid); alkalizing agents (such as ammonium hydroxide, ammonium carbonate, potassium hydroxide, sodium bicarbonate, sodium borate, sodium carbonate, diethanolamine, sodium hydroxide and trolamine); antimicrobials (such as benzoic acid, benzyl alcohol, benzalkonium chloride, benzethonium chloride, butylparaben, chlorobutanol, chlorocresol, cresol, dehydroacetic acid, ethylparaben, methylparaben, methylparaben sodium, phenol, phenylethyl alcohol, phenylmercuric acetate, phenylmercuric nitrate, potassium benzoate, potassium sorbate, propylparaben, propylparaben sodium, sodium propionate, sorbic acid, sodium benzoate, sodium dehydroacetate, thimerosal and thymol); antioxidants (such as ascorbic acid, ascorbyl palmitate, butylated hydroxyanisole, butylated hydroxytoluene, hypophosphorous acid, monothioglycerol, propyl gallate, sodium metabisulfite, sodium thiosulfate, sulfur dioxide, sodium formaldehyde sulfoxylate and tocopherols); buffering agents (such as acetic acid, ammonium carbonate, ammonium phosphate, boric acid, citric acid, potassium metaphosphate, potassium phosphate monobasic, lactic acid, phosphoric acid, potassium citrate, sodium acetate, sodium citrate, sodium lactate solution, dibasic sodium phosphate, and monobasic sodium phosphate); chelating agents (such as edetate disodium, ethylenediaminetetraacetic acid and edetic acid); coating agents (such as cellulose acetate, cellulose acetate phthalate, ethylcellulose, gelatin, hydroxypropyl cellulose, hydroxypropyl methylcellulose, hydroxypropyl methylcellulose, methacrylic acid copolymer, methylcellulose, polyethylene glycol, polyvinyl acetate phthalate, phthalate, sodium carboxymethylcellulose, shellac, sucrose, titanium dioxide, carnauba wax and microcrystalline wax); colorants (such as caramel, red, yellow, black and ferric oxide); complexing agents (such as ethylenediaminetetraacetic acid (EDTA), edetic acid, gentisic acid ethanolamide and oxyquinoline sulfate); diluents (such as calcium carbonate, dibasic calcium phosphate, tribasic calcium phosphate, calcium sulfate, microcrystalline cellulose, powdered cellulose, dextrates, dextrin, dextrose excipient, fructose, kaolin, lactose, mannitol, sorbitol, starch, pregelatinized starch, sucrose, compressible sugar and confectioner's sugar); disintegrants (such as alginic acid, microcrystalline cellulose, croscarmellose sodium, crospovidone, polacrilin potassium, sodium starch glycolate, starch, pregelatinized starch); desiccants (such as calcium chloride, calcium sulfate, and silicon dioxide); solubilizing agents (such as acacia, cholesterol, glyceryl monostearate, diethanolamine, lanolin alcohols, lecithin, mono- and diglycerides, monoethanolamine, oleic acid, oleyl alcohol, poloxamer, polyoxyethylene 50 stearate, polyoxyl 35 castor oil, polyoxyl 40 hydrogenated castor oil, polyoxyl 10 oleyl ether, polyoxyl 20 cetostearyl ether, polyoxyl 40 stearate, polysorbate 20, polysorbate 40, polysorbate 60, polysorbate 80, propylene glycol diacetate, propylene glycol monostearate, sodium lauryl sulfate, sodium stearate, sorbitan monolaurate, sorbitan monooleate, sorbitan monopalmitate, sorbitan monostearate and stearic acid); plasticizers (such as castor oil, diacetylated monoglycerides, diethyl phthalate, glycerin, mono- and di-acetylated monoglycerides, polyethylene glycol, propylene glycol, triacetin and triethyl citrate); polymers (such as cellulose acetate, alkyl celluloses, hydroxyalkyl cellulose, acrylic polymers and copolymers); sorbents (such as powdered cellulose, charcoal and purified siliceous earth); carbon dioxide sorbents (such as barium hydroxide lime and soda lime); stiffening agents (such as hydrogenated castor oil, cetostearyl alcohol, cetyl alcohol, cetyl esters wax, hard fat, paraffin, polyethylene excipient, stearyl alcohol, emulsifying wax, white wax and yellow wax); suspending and/or viscosity-increasing agents (such as acacia, agar, alginic acid, aluminum monostearate, bentonite, purified bentonite, magma bentonite, carbomer, carboxymethylcellulose calcium, carboxymethylcellulose sodium, carboxymethylcellulose sodium 12, carrageenan, microcrystalline and carboxymethylcellulose sodium cellulose, dextrin, gelatin, guar gum, hydroxyethyl cellulose, hydroxypropyl cellulose, hydroxypropyl methylcellulose, magnesium aluminum silicate, methylcellulose, pectin, polyethylene oxide, polyvinyl alcohol, povidone, propylene glycol alginate, silicon dioxide, colloidal silicon dioxide, sodium alginate, tragacanth gum and xanthan gum); lubricants (such as calcium stearate, glyceryl behenate, magnesium stearate, light mineral oil, polyethylene glycol, sodium stearyl fumarate, stearic acid, purified stearic acid, talc, hydrogenated vegetable oil and zinc stearate); and/or tonicity agents (such as dextrose, glycerin, mannitol, potassium chloride and sodium chloride). This is a nonexclusive list and merely represents classes of excipients that may be used in the oral dosage forms as described herein.

The inventors have discovered that the ratio between the monomer and solvent in fluid fill mass, between MAA and PEG, is an important parameter of the present invention. In the invention, the ratio between the monomer and the solvent, in particular between MAA and PEG, is in the range between 20:80 and 95:5, more preferably in the range between 30:70 and 90:10.

In another preferred embodiment of the invention, the ratio between the monomer and the solvent, in particular between MAA and PEG, is in the range between 35:65 and 50:50, preferably in the range between 35:65 and 45:55 and more preferably in the range between 35:65 and 40:60.

In another preferred embodiment of the invention, the ratio between the monomer and the solvent, in particular between MAA and PEG, is in the range between 60:40 and 90:10, preferably between 70:30 and 90:10, and more preferably in the range between 80:20 and 90:10.

### Methods of Use

The embodiments of the present disclosure also include methods of delivering active ingredients to a subject. These include naproxen, acetylsalicylic acid, ibuprofen and acetaminophen or their salt forms.

The embodiments are also useful for delivering active ingredients to a subject that are useful in treating, for example: rheumatoid arthritis (including juvenile rheumatoid arthritis or Still's disease), ankylosing spondylitis, and osteo-arthritis, fibrositis, capsulitis, bursitis, tendinitis, tenosynovitis, and post-episiotomy pain and post-partum pain. Additional methods include, for example, to treat cough and cold, allergy, and to temporarily reduce fever, or to deliver active ingredients such as analgesics and anti-inflammatories to treat treat minor aches and pains due to, for example, soft-tissue injuries such as sprains and strains, arthritic pain, backache, headache, muscular aches, menstrual cramps, and toothache.

One preferred method of use is in the treatment of pain or discomfort that may arise hours after oral administration. Some formulations, for example, can be administered in the evening, prior to going to sleep, but API release from these formulations is delayed so that the peak activity of the API occurs in the hours prior to and during the waking period. Such formulations have a longer Cₘₐₓ and Tₘₐₓ, thus achieving a delayed time till peak activity and sustained levels of API in plasma. In this manner, the formulations can relieve or prevent discomfort or pain predicted to arise 2-10, or preferably 2-6 hours after administration and sustains activity for hours beyond that period.

### Preferred Embodiments of the Fluid Fill Mass

In one preferred embodiment, the dosage form comprises:
a. an active ingredient, chosen from the group consisting of naproxen, acetylsalicylic acid, ibuprofen and acetaminophen or their salt forms,
b. polymerizable monomers selected from methacrylic acid,
c. solvents selected from polyethylene glycol, and
d. a photoinitiator is selected from the list consisting of 2,2-dimethoxy-2-phenylacetophenone, α-hydroxy-ketones and benzophenones derivatives.

In another preferred embodiment, the dosage form, preferably the pharmaceutical dosage form, comprises:
a. A mould or a shell comprising a softgel; and
b. A fluid fill mass inside the softgel shell of a), wherein the fluid fill mass comprises acetylsalicylic acid, a solvent and a polymerizable monomer, wherein the ratio between the monomer and the solvent, MAA and PEG, is in the range between 20:80 and 95:5, preferably in the range between 30:70 and 90:10, and wherein the fluid fill mass inside the softgel shell does not comprise a cross linking agent. Preferably, the fluid fill mass contains a co-solvent such as ethanol.

In another preferred embodiment, the dosage form, preferably the pharmaceutical dosage form, comprises:
a. A mould or a shell comprising a softgel; and
b. A fluid fill mass inside the softgel shell of a), wherein the fluid fill mass comprises acetylsalicylic acid, a solvent and a polymerizable monomer, wherein the ratio between the monomer and the solvent, MAA and PEG, is in the range between 35:65 and 50:50, preferably in the range between 35:65 and 45:55 and more preferably in the range between 35:65 and 40:60, and wherein the fluid fill mass inside the softgel shell does not comprise a cross linking agent. Preferably, the fluid fill mass contains a co-solvent such as ethanol.

In another preferred embodiment, the dosage form, preferably the pharmaceutical dosage form, comprises:
a. A mould or a shell comprising a softgel; and
b. A fluid fill mass inside the softgel shell of a), wherein the fluid fill mass comprises acetylsalicylic acid, a solvent and a polymerizable monomer, wherein the ratio between the monomer and the solvent, MAA and PEG, is in the range between 60:40 and 90:10, preferably between 70:30 and 90:10, and more preferably in the range between 80:20 and 90:10, and wherein the fluid fill mass inside the softgel shell does not comprise a cross linking agent. Preferably, the fluid fill mass contains a co-solvent such as ethanol.

In another preferred embodiment, the dosage form, preferably the pharmaceutical dosage form, comprises:
a. A mould or a shell comprising a softgel; and
b. A fluid fill mass inside the softgel shell of a), wherein the fluid fill mass comprises 35.5 % by weight of acetylsalicylic acid, 57.5 % by weight of methacrylic acid, 5.4 % by weight of polyethylene glycol, 1.0 % by weight of ethanol and 0.6 % by weight of 2,2-dimethoxy-2-phenylacetophenone, and wherein the fluid fill mass encapsulated inside the softgel shell does not comprise a cross linking agent.

In another preferred embodiment, the dosage form, preferably the pharmaceutical dosage form, comprises:
a. A mould or a shell comprising a softgel; and
b. A fluid fill mass inside the softgel shell of a), wherein the fluid fill mass comprises naproxen sodium, a solvent and a polymerizable monomer, wherein the ratio between the monomer and the solvent, MAA and PEG, is in the range between 20:80 and 95:5, preferably in the range between 25:75 and 90:10, more preferably in the range between 30:70 and 70:30, more preferably between 35:65 and 50:50, and wherein the fluid fill mass inside the softgel shell does not comprise a cross linking agent. Preferably, the fluid fill mass contains a photo initiator such as 2,2-dimethoxy-2-phenylacetophenone.

In another preferred embodiment, the dosage form, preferably the pharmaceutical dosage form, comprises:
a. A mould or a shell comprising a softgel; and
b. A fluid fill mass inside the softgel shell of a), wherein the fluid fill mass comprises naproxen sodium, a solvent and a polymerizable monomer, wherein the ratio between the monomer and the solvent, MAA and PEG, is in the range between 35:65 and 45:55 and preferably in the range between 35:65 and 40:60, and wherein the fluid fill mass inside the softgel shell does not comprise a cross linking agent. Preferably, the fluid fill mass contains a photo initiator such as 2,2-dimethoxy-2-phenylacetophenone.

In another preferred embodiment, the dosage form, preferably the pharmaceutical dosage form, comprises:
a. A shell comprising a softgel; and
b. A fluid fill mass inside the softgel shell of a), wherein the fluid fill mass, comprises 27.4 % by weight of naproxen sodium, 25.3 % by weight of methacrylic acid, 47.0 % by weight of polyethylene glycol and 0.3 % by weight of 2,2-dimethoxy-2-phenylacetophenone, and wherein the fluid fill mass encapsulated inside the softgel shell does not comprise a cross linking agent.

In another preferred embodiment, the dosage form, preferably the pharmaceutical dosage form, comprises:
a. A mould or a shell comprising a softgel; and
b. A fluid fill mass inside the softgel shell of a), wherein the fluid fill mass comprises ibuprofen, a solvent and a polymerizable monomer, wherein the ratio between the monomer and the solvent, MAA and PEG, is in the range between 20:80 and 95:5, preferably in the range between 25:75 and 90:10, more preferably in the range between 30:70 and 70:30, more preferably between 35:65 and 50:50.

In another preferred embodiment, the dosage form, preferably the pharmaceutical dosage form, comprises:
a. A mould or a shell comprising a softgel; and
b. A fluid fill mass inside the softgel shell of a), wherein the fluid fill mass comprises ibuprofen, a solvent and a polymerizable monomer, wherein the ratio between the monomer and the solvent, MAA and PEG, is in the range between 35:65 and 45:55 and preferably in the range between 35:65 and 40:60, and wherein the fluid fill mass inside the softgel shell does not comprise a cross linking agent. Preferably, the fluid fill mass contains a photo initiator such as 2,2-dimethoxy-2-phenylacetophenone.

In another preferred embodiment, the dosage form, preferably the pharmaceutical dosage form, comprises:
a. A shell comprising a softgel; and
b. A fluid fill mass inside the softgel shell of a), wherein the fluid fill mass, comprises 42.8% by weight of ibuprofen, 20.0 % by weight of methacrylic acid, 37.0 % by weight of ethanol and 0.2 % by weight of 2,2-dimethoxy-2-phenylacetophenone, and wherein the fluid fill mass encapsulated inside the softgel shell does not comprise a cross linking agent.

In another preferred embodiment, the dosage form, preferably the pharmaceutical dosage form, comprises:
a. A mould or a shell comprising a softgel; and
b. A fluid fill mass inside the softgel shell of a), wherein the fluid fill mass comprises a medicament or supplement, a solvent and a polymerizable monomer, wherein the ration between the monomer and the solvent, MAA and PEG or any other solvent mentioned, is in the range between 20:80 and 95:5, preferably in the range between 25:75 and 90:10, more preferably in the range between 30:70 and 70:30, more preferably between 35:65 and 50:50, and wherein the fluid fill mass encapsulated inside the softgel shell does not comprise a cross linking agent. Preferably, the fluid fill mass contains a co-solvent;
wherein the medicament is acetaminophen.

In another preferred embodiment, the dosage form, preferably the pharmaceutical dosage form, comprises:
a. A mould or a shell comprising a softgel; and
b. A fluid fill mass inside the softgel shell of a), wherein the fluid fill mass comprises a medicament or supplement, a solvent and a polymerizable monomer, wherein the ration between the monomer and the solvent, MAA and PEG, is in the range between 35:65 and 45:55 and preferably in the range between 35:65 and 40:60 and wherein the fluid fill mass encapsulated inside the softgel shell does not comprise a cross linking agent. Preferably, the fluid fill mass contains a co-solvent;
wherein the medicament is acetaminophen.

Another embodiment of the present invention, refers to a dosage form, preferably a pharmaceutical dosage form, obtained or obtainable by irradiating the fluid fill mass of any preferred embodiments, with an UV-Vis lamp, optionally for a few minutes, typically between 2-20 min, to provide a solid or gel-like polymeric fill matrix inside the mould of "a." above. Alternatively, the embodiment of the invention refers to a dosage form, preferably a pharmaceutical dosage form, comprising a polymeric fill matrix that entraps the active ingredients. Said polymeric fill matrix comprises an active ingredient with a concentration between 1 to 70% weight/weight; the concentration of polymers derived from the polymerization of the monomers lies between 10 to 99% weight/weight; and the concentration of solvents lies between 0 to 89% weight/weight. It is noted, that the dosage form according to this embodiment, may or not comprise the solid or semi-solid polymeric fill mass inside a mould, in this sense, the mould can be discarded after the polymerization process.

Not part of the invention, the dosage form, preferably the pharmaceutical dosage form, comprises a fluid fill mass comprising:
a. naproxen or its salt form;
b. methacrylic acid;
c. sodium methacrylate;
d. polyethylene glycol (PEG);
e. water
f. ethanol; and
g. DMPA.

Not part of the invention, the dosage form, preferably the pharmaceutical dosage form, comprises a fluid fill mass comprising:
a. naproxen sodium or naproxen in an amount between about 18% and about 27% by weight;
b. methacrylic acid in an amount between about 7% and about 41% by weight;
c. sodium methacrylate in an amount between about 0% and about 20% by weight;
d. PEG 400 in an amount between about 14% and about 53% by weight;
e. purified water in an amount between about 0% and about 37% by weight;
f. ethanol in an amount between about 0% and about 37% by weight; and
g. DMPA in an amount less than about 1% by weight.

In another preferred embodiment, the dosage form, preferably the pharmaceutical dosage form, comprises a fluid fill mass comprising:
a. naproxen sodium in the amount of about 18% by weight;
b. methacrylic acid in the amount of about 28% to about 41% by weight;
c. PEG 400 between about 40% and about 53% by weight; and
d. DMPA in an amount less than about 1% by weight.

Not part of the invention, the dosage form, preferably the pharmaceutical dosage form, comprises a fluid fill mass comprising:
e. naproxen sodium or naproxen in the amount of about 25%-29% by weight;
f. sodium methacrylate in the amount of about 10-15% by weight;
g. methacrylic acid in the amount of about 10-15% by weight;
h. PEG 400 between about 22% and about 25% by weight;
i. water between about 22% and about 25% by weight; and
j. DMPA in an amount less than about 1% by weight.

Not part of the invention, the dosage form, preferably the pharmaceutical dosage form, comprises a fluid fill mass comprising:
k. naproxen sodium or naproxen in the amount of about 25%-29% by weight;
l. a mixture of sodium methacrylate and/or methacrylic acid totalling about 22-25% by weight, the mixture containing sodium methacrylate in the amount of about 0-25% by weight and methacrylic acid in the amount of about 0-25% by weight;
m. PEG 400 between about 22% and about 25% by weight; and
n. DMPA in an amount less than about 1% by weight.

In another preferred embodiment, the dosage form, preferably the pharmaceutical dosage form, comprises:
a. naproxen or its salt form;
b. methacrylic acid;
c. PEG 400
d. DMPA; and
e. Povidone K12.

In another preferred embodiment, the dosage form, preferably the pharmaceutical dosage form, comprises:
a. between about 2% and about 15% by weight of naproxen acid;
b. between about 28% and about 48% by weight of methacrylic acid;
c. between about 40% and about 62% by weight of PEG 400;
d. less than about 1% by weight of DMPA; and
e. between about 2% and about 5% of Povidone K12.

In another preferred embodiment, the dosage form, preferably the pharmaceutical dosage form, comprises:
a. between about 12% and about 18% by weight of naproxen acid;
b. between about 25% and about 35% by weight of methacrylic acid;
c. between about 50% and about 65% by weight of PEG 400;
d. less than about 1% by weight of DMPA; and
e. between about 1% and about 8% of Povidone K12.

In another preferred embodiment, the dosage form, preferably the pharmaceutical dosage form, comprises a polymeric fill matrix comprising:
a. naproxen sodium in an amount between about 18% and about 27% by weight;
b. polymer comprising methacrylic acid polymer subunits in an amount between about 20% and about 45% by weight;
c. PEG 400 in an amount between about 14% and about 53% by weight;
d. purified water in an amount between about 0% and about 37% by weight;
e. ethanol in an amount between about 0% and about 37% by weight; and
f. residual compounds resulting from a photoinitiation process in an amount less than about 1% by weight.

In another preferred embodiment, the invention comprises treating a subject with the above formulation.

In another preferred embodiment, the dosage form, preferably the pharmaceutical dosage form comprises a polymeric fill matrix comprising:
a. naproxen sodium in an amount between about 18% and about 28% by weight;
b. polymer comprises methacrylic acid polymer subunits in an amount between about 28% and about 41% by weight;
c. PEG 400 in an amount between about 40% and about 52% by weight; and
d. residual compounds resulting from a photoinitiation process in an amount less than about 1% by weight.
In another preferred embodiment, the invention comprises treating a subject with the above formulation.

In another preferred embodiment, the dosage form, preferably the pharmaceutical dosage form comprises a polymeric fill matrix comprising:
a. naproxen sodium in the amount of about 24%-30% by weight;
b. polymer comprises methatcrylic acid polymer subunits in the amount of about 20-30% by weight;
c. PEG 400 between about 20% and about 30% by weight;
d. water between about 20% and about 30% by weight; and
e. residual compounds resulting from a photoinitiation process in an amount less than about 1% by weight.

In another preferred embodiment, the invention comprises treating a subject with the above formulation.

Not part of the invention, the dosage form, preferably the pharmaceutical dosage form comprises a polymeric fill matrix comprising:
a. naproxen sodium or naproxen in the amount of about 25%-29% by weight;
b. a mixture of methacrylic acid polymers and/or sodium methacrylate polymers that contains methacrylic acid polymer in an amount between about 0% and about 30% by weight and sodium methacrylate polymer in an amount between about 0% and about 30% by weight;
c. PEG 400 between about 22% and about 25% by weight; and
d. residual compounds resulting from a photoinitiation process in an amount less than about 1% by weight.

In another preferred embodiment, the invention comprises treating a subject with the above formulation.

In another preferred embodiment, the dosage form, preferably the pharmaceutical dosage form comprises a polymeric fill matrix comprising:
a. naproxen acid in the amount between about 2% and about 15% by weight;
b. polymer comprises methatcrylic acid polymer subunits in the amount of between about 28% and about 50% by weight;
c. PEG 400 in the amount between about 40% and about 65% by weight;
d. Povidone K12 in the amount between about 2% and about 6% by weight; and
e. residual compounds resulting from a photoinitiation process in an amount less than about 1% by weight.

In another preferred embodiment, the invention comprises treating a subject with the above formulation.

In another preferred embodiment, the dosage form, preferably the pharmaceutical dosage form comprises a polymeric fill matrix comprising:
a. naproxen acid in the amount between about 14% and about 16% by weight;
b. polymer comprises methatcrylic acid polymer subunits in the amount between about 28% and about 33% by weight
c. PEG 400 in the amount between about 52% and about 62% by weight;
d. Povidone K12 in the amount between about 2% and about 5% by weight; and
e. residual compounds resulting from a photoinitiation process in an amount less than about 1% by weight.

In another preferred embodiment, the invention comprises treating a subject with the above formulation.

In another preferred embodiment, the fluid fill mass is optionally fully homogenized at a temperature from 25 to 40 °C, under stirring and protected against light, before the irradiation step.

Another embodiment of the invention refers to a method for manufacturing dosage forms, preferably pharmaceutical dosage forms, based in soft gel capsules with a polymeric fill matrix entrapping active ingredients, wherein the process comprises the following steps:
a. Providing a fluid fill mass, wherein the fill mass comprises an active ingredient or with a concentration comprised between 1% to 70% weight/weight, polymerizable monomers with a concentration comprised between 10 to 99% weight/weight, solvents with a concentration comprised between 0 to 89% weight/weight and UV-Vis photoinitiator, to allow the free-radical photopolymerization of the monomers, with concentration comprised between 0.05 to 5 % weight/weight wherein the amount of the UV-Vis photoinitiator is from 0 to 5% weight/weight if autopolymerizables monomers are used; and
b. Encapsulating said fluid fill mass of step a) within a softgel capsule or within a suitable mould prior to polymerization.

In a preferred embodiment of the manufacturing method, the process further comprises irradiating the product of b) with a UV-Vis lamp, optionally for a few minutes, typically between 2-20 min, to provide a solid or semi-solid polymeric fill matrix encapsulated inside the softgel shell of b).

Suitable photopolymerization is obtained when wavelength of maximum absorption of photoinitiator is similar to maximum emission of UV-lamp. The polymerization process depends on curing depth, light intensity and photoinitiator concentration. These variables should be adapted based on photoinitiator selected. In addition, the polymerization process may be initiated in the tumble dryer thereby significantly reducing the curing time, resources, energy, and personnel necessary to dry and cure the capsules of the present invention when compared to the prior art. In the present invention, the drying process takes a few minutes, compared to the prior art process, which requires approximately 4-6 days.

In another preferred embodiment of the invention, the fluid fill mass is optionally fully homogenized at a temperature from 25 to 40 °C, under stirring and protected against exposure to light at certain wavelengths, before the irradiation step.

In yet another preferred embodiment of the manufacturing method, the active ingredients, the polymerizable monomers and the solvents used to produce the dosage forms of the invention are as illustrated in any of the prior embodiments.

The following examples are only for illustrative purposes.

### COMPARISON BETWEEN FRUTOS ET AL. AND EMBODIMENTS OF THE INVENTION

Table 1 below shows specific embodiments of the present invention used for comparison with naproxen formulations disclosed in Frutos *et al.*

**Table 1:**

| | **Frutos (HCPK)** | **Frutos (DMPA)** | **Frutos (DMPA & PEGMEMA)** | **Frutos (HCPK & PEGMEMA)** | **Naproxen 1 (DMPA)** | **Naproxen 2 (HCPK)** | **Naproxen 3 (Solvent)** |
|---|---|---|---|---|---|---|---|
| | **(%)** | **(%)** | **(%)** | **(%)** | **(%)** | **(%)** | **(%)** |
| **Photoinitiator (HCPK)** | 1.0 | 0.0 | 0.0 | 1.0 | 0.0 | 1.0 | 0.0 |
| **Photoinitiator (DMPA)** | 0.0 | 1.0 | 1.0 | 0.0 | 1.0 | 0.0 | 1.0 |
| **Cross-linker (TEGDMA)** | 1.0 | 1.0 | 0.0 | 0.0 | 0.0 | 0.0 | 0.0 |
| **Naproxen sodium** | 18.3 | 18.3 | 18.3 | 18.3 | 18.3 | 18.3 | 18.3 |
| **Monomers (MAA:PEGMEMA)** | 50.0 | 50.0 | 80.7 | 80.7 | 0.0 | 0.0 | 0.0 |
| **Monomers (MAA:PEG)** | 0.0 | 0.0 | 0.0 | 0.0 | 80.7 | 80.7 | 72.9 |
| **Solvent EtOH:H2O** | 29.7 | 29.7 | 0.0 | 0.0 | 0.0 | 0.0 | 7.8 |
| **Total** | 100.0 | 100.0 | 100.0 | 100.0 | 100.0 | 100.0 | 100.0 |

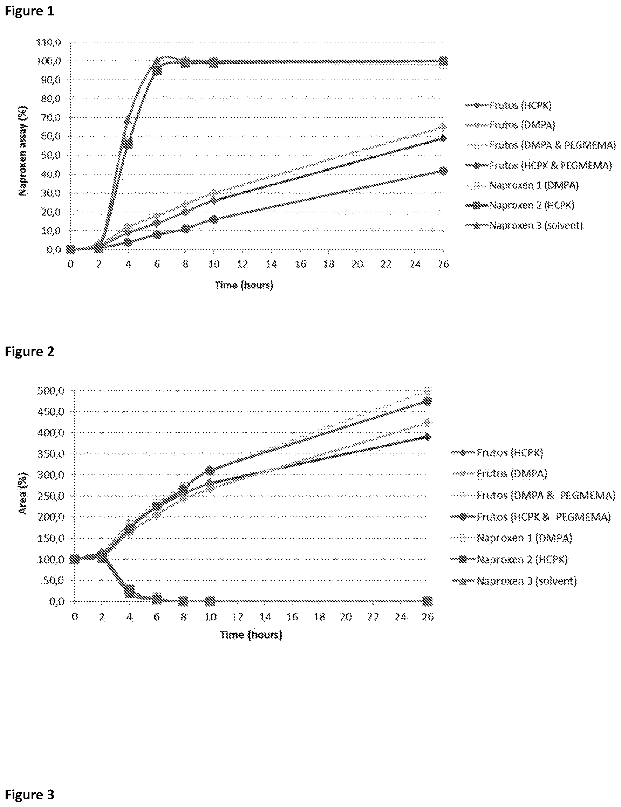

The above formulations were tested for dissolution at different pH conditions as revealed in Figure 1. For the dissolution testing, capsules were incubated in solution at pH 1.2 for 2 hours, followed by a shift to pH 7.4 for the remainder [method based on dissolution medium described in USP40-NF35].

The inventors discovered that by eliminating cross-linking agents and changing monomer PEGMEMA to solvent PEG they could achieve formulations suitable for human consumption. The naproxen dissolution profiles disclosed in Figure 1 clearly demonstrate that these embodiments provide drug dissolution profiles that are suitable for immediate release formulations. The Frutos *et al.* formulations, by comparison, are not.

The inventors also compared the swelling behaviour of the Frutos *et al.* formulations with the above embodiments of the present invention measuring capsule size. This is seen in Figure 2, which demonstrates that the Frutos *et al.* formulations exhibit swelling behaviour that shows excessive volume increase due to intake of the dissolution media by the polymeric fill matrix. The embodiments of the invention, meanwhile, do not exhibit the same swelling characteristics.

In combination, these data demonstrate that the tested embodiments of the present invention rapidly dissolve, while the formulations provided by Frutos *et al.* are both unsuitable for rapid dissolution and swell excessively in conditions simulating those in the GI tract, which makes the latter formulation unsuitable for human intake.

### EXAMPLES

The below examples provide specific embodiments of the present invention. The specific embodiments show exemplary formulations containing such APIs as naproxen, acetylsalicylic acid (ASA) and ibuprofen, but the use of these specific APIs in these examples does not limit the invention.

Solubility of actives was evaluated considering range of pH in GI tract (1 - 8), as provided by EMEA specifications. *See, e.g.,* "Guideline on quality of oral modified release products," MA/CHMP/QWP/428693(2013).

### Example 1. Formulations containing naproxen

Naproxen is a non-steroidal anti-inflammatory drug used as an analgesic. The minimum effective dose is 200 mg. In the case of being formulated as sodium salt (naproxen sodium), the minimum effective dose would be 220 mg. The chemical structure of each is shown below.

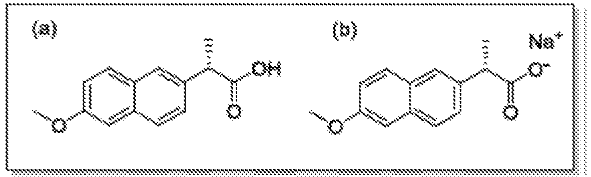

### Chemical structure of naproxen (a) and naproxen sodium (b).

Formulations described in Table 2 below provide embodiments of the present invention that use 220 mg of naproxen sodium. Notably, none contain PEGMEMA monomer.

**Table 2:**

| | **Naproxen 1 (DMPA)** | **Naproxen 2 (HCPK)** | **Naproxen 3 (solvent)** |
|---|---|---|---|
| | **(%)** | **(%)** | **(%)** |
| **Photoinitiator (HCPK)** | 0.0 | 1.0 | 0.0 |
| **Photoinitiator (DMPA)** | 1.0 | 0.0 | 1.0 |
| **Cross-linker (TEGDMA)** | 0.0 | 0.0 | 0.0 |
| **Naproxen sodium** | 18.3 | 18.3 | 18.3 |
| **Monomers (MAA:PEG)** | 80.7 | 80.7 | 72.9 |
| **Solvent EtOH:H2O** | 0.0 | 0.0 | 7.8 |
| **Total** | 100.0 | 100.0 | 100.0 |

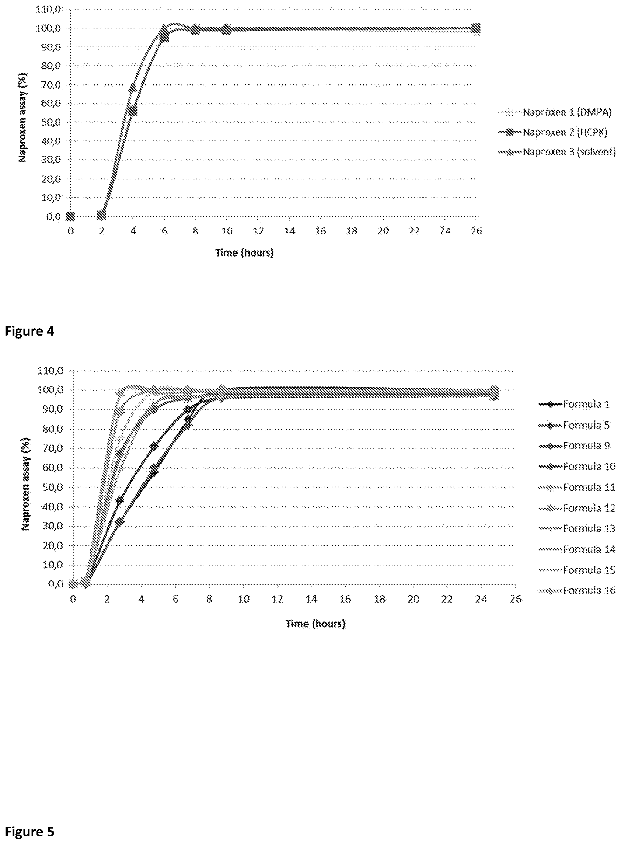

Figure 3 provides dissolution profiles for the formulations listed above. The figure shows incubation for 2 hours at pH 1.2, followed by a shift to pH 7.4 for the remainder of the test [method based on dissolution medium described in USP40-NF35]. As revealed in the figure, approximately 100% of the naproxen was released within 4 hours of the pH shift for all formulations.

Additional embodiments of the present invention using 220 mg of naproxen sodium are provided in Table 3 below, which discloses the modification of MAA monomer to its sodium salt form, sodium methacrylate (MAA⁻ Na⁺), and varying the solvent ratios.

**Table 3: (formulations 5-16 are reference examples).**

| | **Formula 1 (%)** | **Formula 5 (%)** | **Formula 9 (%)** | **Formula 10 (%)** | **Formula 11 (%)** | **Formula 12 (%)** | **Formula 13 (%)** | **Formula 14 (%)** | **Formula 15 (%)** | **Formula 16 (%)** |
|---|---|---|---|---|---|---|---|---|---|---|
| **Naproxen sodium** | 27.4 | 27.4 | 27.4 | 27.4 | 21.94 | 21.94 | 18.28 | 18.28 | 18.28 | 18.28 |
| **Methacrylic acid** | 25.3 | 17.7 | 12.7 | 7.0 | 13.61 | 8.17 | 14.25 | 8.55 | 8.55 | 8.55 |
| **Sodium methacrylate** | 0.0 | 7.6 | 12.7 | 17.7 | 13.61 | 19.06 | 14.25 | 19.95 | 19.95 | 19.95 |
| **PEG 400** | 47.0 | 32.9 | 23.5 | 14.1 | 25.28 | 15.17 | 26.47 | 15.88 | 26.47 | 37.05 |
| **Purified water** | 0.0 | 14.1 | 23.5 | 32.9 | 25.28 | 35.39 | 26.47 | 37.05 | 26.47 | 15.88 |
| **DMPA** | 0.3 | 0.3 | 0.3 | 0.3 | 0.27 | 0.27 | 0.29 | 0.29 | 0.29 | 0.29 |
| **Total** | 100.0 | 100.0 | 100.0 | 100.0 | 100.0 | 100.0 | 100.0 | 100.0 | 100.00 | 100.00 |

Figure 4 provides dissolution profiles for the formulations listed above. For the dissolution testing, capsules were incubated in 900 mL solution at pH 1.2 (0.1 N HCl) for 45 minutes at 37°C, using paddle apparatus at 50 rpm followed by a shift to pH 6.2 by adding 100 mL of sodium phosphate buffer solution (consisting of 100.0 g of sodium phosphate tribasic dodecahydrate plus 17.0 g of NaOH in 1000 mL of purified water) at 37°C , for 24 hours maintaining dissolution test conditions. The figure shows that within 4 hours of the pH shift approximately 100% of the naproxen is released from the softgel. Modifications tested impact on release of naproxen in the GI tract. Substitution of MAA to its salt form reduces the degree of physical crosslinking and thus dissolution of polymer matrix is favored. These results provide multiple polymer fill matrix formulations that are suitable for human intake and that dissolve quickly in the GI.

Embodiments of the present invention containing 200 mg of the acid form of naproxen are provided in Table 4 below.

**Table 4**

| | **F05816-1 (%)** | **F05816-4 (%)** | **F05816-5 (%)** | **F05816-8 (%)** |
|---|---|---|---|---|
| **MAA** | 46.1 | 40.1 | 47.3 | 41.1 |
| **PEG 400** | 46.1 | 40.1 | 47.3 | 41.1 |
| **DMPA** | 0.5 | 0.4 | 0.5 | 0.4 |
| **Povidone K12** | 4.8 | 4.8 | 2.5 | 2.5 |
| **Naproxen acid** | 2.4 | 14.5 | 2.5 | 14.9 |
| **Total** | 100.0 | 100.0 | 100.0 | 100.0 |

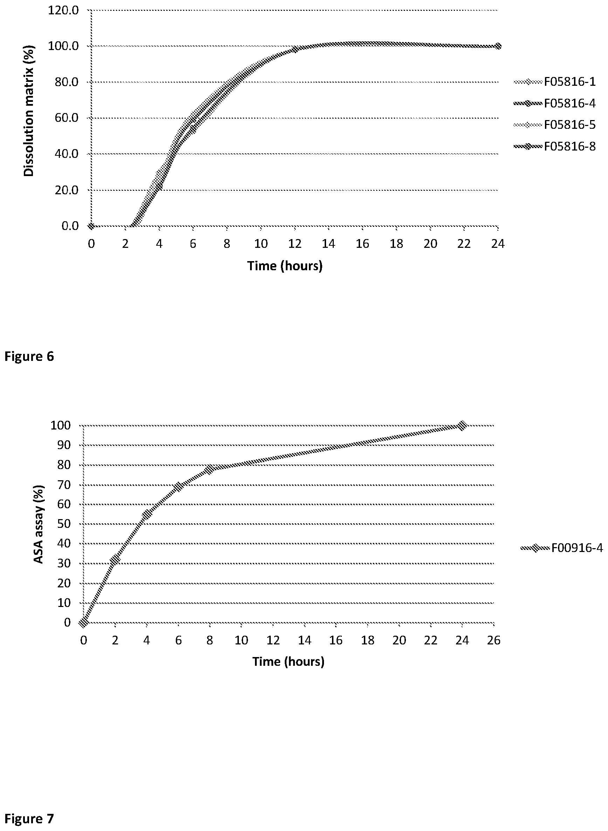

The above formulations all resulted in capsules containing a polymeric matrix where the drug is entrapped. Figure 5 provides dissolution profiles for these formutions. In brief, the capsules were incubated at pH 1.2 for 2 hours, which is raised to 7.4 for the remainder of the test [method based on dissolution medium described in USP40-NF35]. The results show complete dissolution of the Naproxen within 10 hours of the pH shift and demonstrate the suitability of the present invention for generating delayed release or sustained release formulations and for human consumption.

### Example 2. Formulations containing acetylsalicylic acid (ASA).

Different embodiments of the present invention comprising acetylsalicylic acid (ASA) are provided herein. These embodiments include ASA formulations directed to its different therapeutic uses, such as, cardio and pain relief (81, 325, and 500 mg/capsule). Several embodiments of the present invention incorporating ASA were developed and tested for ASA release. Below is an illustration of hydrolysis of ASA to salicylic acid and acetic acid in the presence of water.

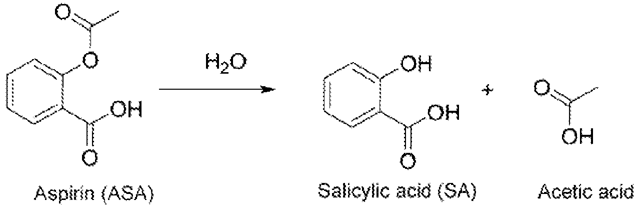

### Conversion of ASA into SA in the presence of water (mole-to-mole reaction).

### 2.1 Formulations containing ASA 81 mg

The next table includes embodiments of the present invention that contain 81 mg of ASA.

**Table 5 (formulations F00816-1 (%) and F00816-2 (%) are reference examples)**

| | **F00716-1 (%)** | **F00716-2 (%)** | **F00716-3 (%)** | **F00816-1 (%)** | **F00816-2 (%)** | **F00916-4 (%)** |
|---|---|---|---|---|---|---|
| **MAA** | 44.1 | 57.8 | 57.8 | 13.2 | 13.2 | 31.1 |
| **Sodium methacrylate** | - | | | 30.9 | 30.9 | |
| **PEG 600** | 47.4 | 31.1 | | | - | 57.8 |
| **PEG 400** | - | - | 31.1 | 23.7 | - | |
| **PEG 350 monomethyl ether** | - | - | - | | 23.7 | - |
| **Purified water** | - | - | - | 23.7 | 23.7 | - |
| **DMPA** | 0.7 | 0.9 | 0.9 | 0.7 | 0.7 | 0.9 |
| **ASA** | 7.8 | 10.2 | 10.2 | 7.8 | 7.8 | 10.2 |
| **Total** | 100.0 | 100.0 | 100.0 | 100.0 | 100.0 | 100.0 |

Each embodiment provided above generated softgels with polymerized fill matrix capable of releasing ASA in physiological conditions. Dissolution testing was performed for each, showing complete dissolution of the polymeric fill matrix and release of the ASA. Dissolution tests are performed at pH 1.2 for 2 hours then pH 6.8 [See Real Farmacopea Española 2008. Monography 2.9.1]. Figure 6 charts the dissolution over time of a representative formulation containing ASA and shows 100% of drug release within 20 hours of commencement.

Tests were conducted to demonstrate the effect of varying the MAA/PEG ratio and the molecular weight of PEG (600 / 400) on the ASA formulations The table below shows the different formulations tested.

**Table 6**

| | **F00916-2 (%)** | **F00916-2B (%)** | **F00916-2C (%)** | **F00916-2D (%)** | **F00916-2E (%)** | **F00916-2F (%)** |
|---|---|---|---|---|---|---|
| **MAA** | 31.1 | 26.7 | 35.7 | 31.1 | 26.7 | 35.7 |
| **PEG 600** | 57.8 | -- | 53.3 | 57.8 | -- | -- |
| **PEG 400** | -- | 62.2 | -- | -- | 62.2 | 53.3 |
| **DMPA** | 0.9 | 0.9 | 0.9 | 0.9 | 0.9 | 0.9 |
| **ASA** | 10.2 | 10.2 | 10.2 | 10.2 | 10.2 | 10.2 |
| **Total** | 100.0 | 100.0 | 100.0 | 100.0 | 100.0 | 100.0 |

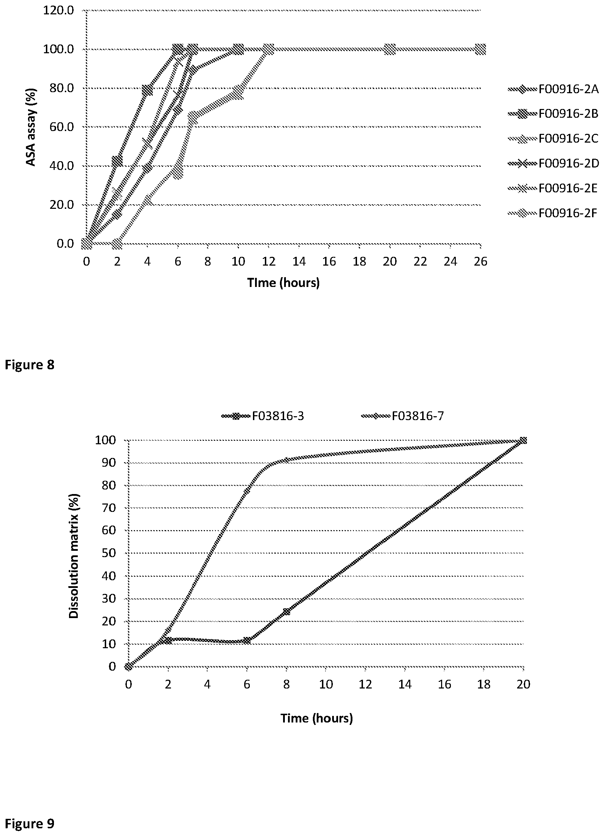

Figure 7 shows the results of dissolution testing on embodiments of the present invention that include ASA and that vary the MAA/PEG ratio and the molecular weight of PEG in the different formulations. Dissolution tests are performed at pH 1.2 for 2 hours then pH 6.8 [See Real Farmacopea Española 2008. Monography 2.9.1]. As revealed in Figure 7, the formulations show varying amounts of drug release in the testing conditions, with all showing complete release within 12 hours of initiating the test.

### 2.2 Formulations containing ASA 500 mg

Embodiments of the present invention containing ASA 500 mg were manufactured and tested. Solf gel capsules within polymeric fill matrix were obtained. The formulations provided in Table 7 below generated.

**Table 7**

| | **F03816-3 (%)** | **F03816-7 (%)** |
|---|---|---|
| **ASA** | 35.6 | 35.6 |
| **MAA** | 32.0 | 22.4 |
| **PEG 400** | 4.8 | 35.4 |
| **Ethanol** | 27.2 | 6.3 |
| **DMPA** | 0.3 | 0.2 |
| **Total** | 100.0 | 100.0 |

Figure 8 shows the dissolution profiles for both formulations. The capsules were incubated at pH 1.2 for two hour followed by incubation at pH 6.8 for the remainder of the test [Real Farmacopea Española 2008. Monograph 2.9.1]. As demonstrated in Figure 8, the inventors discovered that drug release can be controlled by modifying the amount of MAA within the formulation, which makes it possible to generate rapid or controlled release capsules.

### Example 3. Formulations containing ibuprofen.

Ibuprofen is a non-steroidal anti-inflammatory drug commonly used for antipyretic, analgesic and anti-inflammatory purposes. After ingestion, it is absorbed in the upper part of the intestine. Compositions for oral administration contain 600 mg ibuprofen. The structure of ibuprofen is shown below.

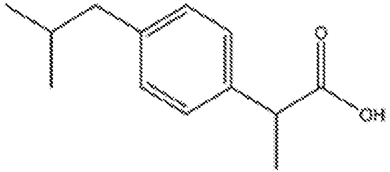

### Chemical structure of ibuprophen.

### Formulations containing ibuprofen

Several embodiments of the invention containing 600 mg of ibuprofen were manufactured and tested. Table 8 provides an example of one such formulation.

**Table 8**

| | **F04616-12 (%)** |
|---|---|
| **Ibuprofen** | 49.9 |
| **MAA** | 17.5 |
| **PEG 400** | 16.2 |
| **KOH** | 16.2 |
| **DMPA** | 0.2 |
| **Total** | 100.0 |

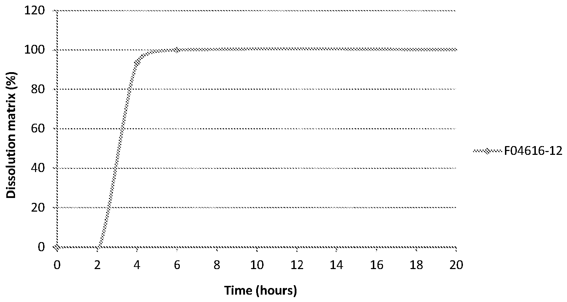

This formulation results in a transparent solid matrix embodiment containing Ibuprofen 600 mg. Figure 9 shows the dissolution profile for this formulation when tested using the protocol applied previously (incubation at pH 1.2 for two hours, followed by incubation at a pH of 6.8 for the remainder of the test) [See Real Farmacopea Española 2008. Monography 2.9.1]. As shown in Figure 9, the capsule releases approximately 100% of the Ibuprofen within 2 hours of shifting the pH, thereby demonstrating the suitability of these embodiments for human use.

## Claims

1. A composition comprising a fluid fill mass comprising:
at least one active ingredient;
at least one polymerizable monomer;
at least one polymerization initiator;
wherein the fluid fill mass does not comprise a cross linking agent and
wherein activation of the polymerization initiator leads to production of a polymeric fill matrix and
wherein at least one active ingredient is chosen from the group consisting of naproxen, acetylsalicylic acid, ibuprofen and acetaminophen or their salt forms and
wherein the monomer is methacrylic acid and the solvent is polyethylene glycol, and wherein the percentage w/w ratio between monomer and solvent in the fluid fill mass is between 20:80 and 95:5.

2. The composition of claim 1, wherein the polymerization initiator is a photoinitiator.

3. The composition of claim 2, wherein the fluid fill mass further comprises: water, diols and alcohols.

4. The composition of claim 1, wherein at least one active ingredient is naproxen or its salt form.

5. The composition of claim 1, wherein at least one active ingredient is acetylsalicylic acid.

6. The composition of claim 1, wherein at least one active ingredient is ibuprofen or its salt form.

7. The composition of claim 1, wherein the fluid fill mass, comprises 27.4 % by weight of naproxen sodium, 25.3 % by weight of methacrylic acid, 47.0 % by weight of polyethylene glycol, and 0.3 % by weight of 2,2-dimethoxy-2-phenylacetophenone.

8. The composition of claim 1, wherein the fluid fill mass comprises 35.5 % by weight of acetylsalicylic acid, 57.5 % by weight of methacrylic acid, 5.4 % by weight of polyethylene glycol, 1.0 % by weight of ethanol and 0.6 % by weight of 2,2-dimethoxy-2-phenylacetophenone.

9. The composition of claim 1, wherein the fluid fill mass, comprises 42.8 % by weight of ibuprofen, 20.0 % by weight of methacrylic acid, 37.0 % by weight of ethanol, and 0.2 % by weight of 2,2-dimethoxy-2-phenylacetophenone.

10. The composition of claim 1, wherein at least one active ingredient is acetaminophen or its salt form.

11. A method of making an oral composition according to claim 1 comprising:
combining at least one active ingredient; at least one polymerizable monomer; and at least one UV-Vis photoinitiator to form a fluid fill mass, wherein the fluid fill mass does not comprise a cross linking agent;
mixing the fluid fill mass to create a homogeneous mixture;
forming the fluid fill mass into the shape of a softgel; and
curing the resulting softgel with UV-Vis light for sufficient time to form a polymeric fill matrix.

12. The method of claim 11 further comprising protecting the combined ingredients from exposure to UV-Vis light from the time that the photoinitiator is added to the fluid fill mass until just before the photoinitiation step.

13. The method of claim 11, wherein the softgel shape is formed by a mould and the mould is sufficiently transparent to UV-Vis light to catalyze photopolymerization.

14. A composition according to claim 1, comprising a fluid fill mass comprising:
naproxen sodium in an amount between about 18% and about 27% by weight;
polymer comprising methacrylic acid polymer subunits in an amount between about 20% and about 45% by weight;
PEG 400 in an amount between about 14% and about 53% by weight;
purified water in an amount between about 0% and about 37% by weight;
ethanol in an amount between about 0% and about 37% by weight; and
residual compounds resulting from a photoinitiation process in an amount less than about 1% by weight.

15. The composition according to claim 14 to deliver active ingredients for use in the treatment of cough and cold, allergy, and to temporarily reduce fever, or for use in the treatment of minor aches and pains due to, for example, arthritic pain, backache, headache, the common cold, muscular aches, menstrual cramps, toothache.

## Patentansprüche

1. Zusammensetzung umfassend eine fluide Füllmasse umfassend:
wenigstens einen Wirkstoff;
wenigstens ein polymerisierbares Monomer;
wenigstens einen Polymerisationsinitiator;
wobei die fluide Füllmasse kein Vernetzungsmittel umfasst, und
wobei Aktivierung des Polymerisationsinitiators zur Erzeugung einer polymeren Füllmatrix führt, und
wobei wenigstens ein Wirkstoff ausgewählt ist der Gruppe bestehend aus Naproxen, Acetylsalicylsäure, Ibuprofen und Acetaminophen oder ihren Salzformen, und
wobei das Monomer Methacrylsäure ist und das Lösungsmittel Polyethylenglycol ist, und wobei der prozentuale w/w-Verhältnis zwischen Monomer und Lösungsmittel in der fluiden Füllmasse zwischen 20:80 und 95:5 beträgt.

2. Zusammensetzung nach Anspruch 1, wobei der Polymerisationsinitiator ein Photoinitiator ist.

3. Zusammensetzung nach Anspruch 2, wobei die fluide Füllmasse ferner umfasst: Wasser, Diole und Alkohole.

4. Zusammensetzung nach Anspruch 1, wobei wenigstens ein Wirkstoff Naproxen oder seine Salzform ist.

5. Zusammensetzung nach Anspruch 1, wobei wenigstens ein Wirkstoff Acetylsalicylsäure ist.

6. Zusammensetzung nach Anspruch 1, wobei wenigstens ein Wirkstoff Ibuprofen oder seine Salzform ist.

7. Zusammensetzung nach Anspruch 1, wobei die fluide Füllmasse 27,4 Gew.-% Naproxen-Natrium, 25,3 Gew.-% Methacrylsäure, 47,0 Gew.-% Polyethylenglycol und 0,3 Gew.-% 2,2-Dimethoxy-2-phenylacetophenon umfasst.

8. Zusammensetzung nach Anspruch 1, wobei die fluide Füllmasse 35,5 Gew.-% Acetylsalicylsäure, 57,5 Gew.-% Methacrylsäure, 5,4 Gew.-% Polyethylenglycol, 1,0 Gew.-% Ethanol und 0,6 Gew.-% 2,2-Dimethoxy-2-phenylacetophenon umfasst.

9. Zusammensetzung nach Anspruch 1, wobei die fluide Füllmasse 42,8 Gew.-% Ibuprofen, 20,0 Gew.-% Methacrylsäure, 37,0 Gew.-% Ethanol und 0,2 Gew.-% 2,2-Dimethoxy-2-phenylacetophenon umfasst.

10. Zusammensetzung nach Anspruch 1, wobei wenigstens ein Wirkstoff Paracetamol oder seine Salzform ist.

11. Verfahren zur Herstellung einer oralen Zusammensetzung nach Anspruch 1, umfassend:
Kombinieren wenigstens eines Wirkstoffs; wenigstens eines polymerisierbaren Monomers; und wenigstens eines UV-Vis-Photoinitiators, um eine fluide Füllmasse zu bilden, wobei die fluide Füllmasse kein Vernetzungsmittel umfasst;
Mischen der fluiden Füllmasse, um ein homogenes Gemisch zu bilden;
Formen der fluiden Füllmasse in die Form eines Weichgels; und
Härten des erhaltenen Weichgels mit UV-Vis-Licht für eine ausreichende Zeit, um eine polymere Füllmatrix zu bilden.

12. Verfahren nach Anspruch 11, ferner umfassend Schützen der kombinierten Inhaltsstoffe vor Exposition gegenüber UV-Vis-Licht ab dem Zeitpunkt, bei dem der Photoinitiator zu der fluiden Füllmasse zugegeben wird, bis unmittelbar vor dem Photoinitiationsschritt.

13. Verfahren nach Anspruch 11, wobei die Weichgelform durch ein Formwerkzeug geformt wird und das Formwerkzeug ausreichend transparent für UV-Vis-Licht ist, um die Photopolymerisation zu katalysieren.

14. Zusammensetzung nach Anspruch 1, umfassend eine fluide Füllmasse umfassend:
Naproxen-Natrium in einer Menge zwischen etwa 18 Gew.-% und etwa 27 Gew.-%;
Polymer umfassend Methacrylsäurepolymer-Untereinheiten in einer Menge zwischen etwa 20 Gew.-% und etwa 45 Gew.-%;
PEG 400 in einer Menge zwischen etwa 14 Gew.-% und etwa 53 Gew.-%;
gereinigtes Wasser in einer Menge zwischen etwa 0 Gew.-% und etwa 37 Gew.-%;
Ethanol in einer Menge zwischen etwa 0 Gew.-% und etwa 37 Gew.-%; und
restliche Verbindungen, die aus einem Photoinitiationsverfahren stammen, in einer Menge von weniger als etwa 1 Gew.-%.

15. Zusammensetzung nach Anspruch 14 zur Abgabe von Wirkstoffen zur Verwendung bei der Behandlung von Husten und Erkältung, Allergie, und zur zeitweiligen Senkung von Fieber, oder zur Verwendung bei der Behandlung von leichtem Schmerz und Schmerz aufgrund von beispielsweise Arthritisschmerz, Rückenschmerz, Kopfschmerz, gewöhnlicher Erkältung, Muskelschmerz, Menstruationskrämpfen, Zahnschmerz.

## Revendications

1. Composition comprenant une masse de remplissage de fluide comprenant :
au moins un ingrédient actif ;
au moins un monomère polymérisable ;
au moins un initiateur de polymérisation ;
dans laquelle la masse de remplissage de fluide ne comprend pas d'agent de réticulation et
dans laquelle l'activation de l'initiateur de polymérisation conduit à la production d'une matrice de remplissage polymère et
dans laquelle au moins un ingrédient actif est choisi dans le groupe constitué par le naproxène, l'acide acétylsalicylique, l'ibuprofène et l'acétaminophène ou leurs formes salines et
dans laquelle le monomère est l'acide méthacrylique et le solvant est le polyéthylène glycol, et dans laquelle le rapport p/p en pourcentage entre le monomère et le solvant dans la masse de remplissage de fluide est compris entre 20:80 et 95:5.

2. Composition selon la revendication 1, dans laquelle l'initiateur de polymérisation est un photoinitiateur.

3. Composition selon la revendication 2, dans laquelle la masse de remplissage de fluide comprend en outre : de l'eau, des diols et des alcools.

4. Composition selon la revendication 1, dans laquelle au moins un ingrédient actif est le naproxène ou sa forme saline.

5. Composition selon la revendication 1, dans laquelle au moins un ingrédient actif est l'acide acétylsalicylique.

6. Composition selon la revendication 1, dans laquelle au moins un ingrédient actif est l'ibuprofène ou sa forme saline.

7. Composition selon la revendication 1, dans laquelle la masse de remplissage de fluide comprend 27,4 % en poids de naproxène sodique, 25,3 % en poids d'acide méthacrylique, 47,0 % en poids de polyéthylèneglycol et 0,3 % en poids de 2,2-diméthoxy-2-phénylacétophénone.

8. Composition selon la revendication 1, dans laquelle la masse de remplissage de fluide comprend 35,5 % en poids d'acide acétylsalicylique, 57,5 % en poids d'acide méthacrylique, 5,4 % en poids de polyéthylèneglycol, 1,0 % en poids d'éthanol et 0,6 % en poids de 2,2-diméthoxy-2-phénylacétophénone.

9. Composition selon la revendication 1, dans laquelle la masse de remplissage de fluide comprend 42,8 % en poids d'ibuprofène, 20,0 % en poids d'acide méthacrylique, 37,0 % en poids d'éthanol et 0,2 % en poids de 2,2-diméthoxy-2-phénylacétophénone.

10. Composition selon la revendication 1, dans laquelle au moins un ingrédient actif est l'acétaminophène ou sa forme saline.

11. Procédé de préparation d'une composition orale selon la revendication 1, comprenant :
la combinaison d'au moins un ingrédient actif ; d'au moins un monomère polymérisable ; et d'au moins un photoinitiateur UV-visible pour former une masse de remplissage de fluide, la masse de remplissage de fluide ne comprenant pas un agent de réticulation ;
le mélange de la masse de remplissage de fluide pour créer un mélange homogène ;
la mise en forme de la masse de remplissage de fluide sous la forme d'un gel souple ; et
le durcissement du gel souple résultant avec une lumière UV-visible pendant une durée suffisante pour former une matrice de remplissage polymère.

12. Procédé selon la revendication 11, comprenant en outre la protection des ingrédients combinés contre une exposition à de la lumière UV-visible à partir du moment où le photoinitiateur est ajouté à la masse de remplissage de fluide jusqu'à juste avant l'étape de photoinitiation.

13. Procédé selon la revendication 11, dans lequel la forme de gel souple est mise en forme par un moule et le moule est suffisamment transparent à de la lumière UV-visible pour catalyser une photopolymérisation.

14. Composition selon la revendication 1, comprenant une masse de remplissage de fluide comprenant :
du naproxène sodique en une quantité comprise entre environ 18 % et environ 27 % en poids ;
un polymère comprenant des sous-unités de polymère d'acide méthacrylique en une quantité comprise entre environ 20 % et environ 45 % en poids ;
du PEG 400 en une quantité comprise entre environ 14 % et environ 53 % en poids ;
de l'eau purifiée en une quantité comprise entre environ 0 % et environ 37 % en poids ;
de l'éthanol en une quantité comprise entre environ 0 % et environ 37 % en poids ; et
des composés résiduels résultant d'un procédé de photoinitiation en une quantité inférieure à environ 1 % en poids.

15. Composition selon la revendication 14 pour délivrer des ingrédients actifs pour une utilisation dans le traitement de la toux et du rhume, des allergies, et pour réduire temporairement la fièvre, ou pour une utilisation dans le traitement de douleurs mineures dues, par exemple, à une douleur arthritique, à un mal de dos, à un mal de tête, à un rhume commun, à des douleurs musculaires, à des crampes menstruelles, à des maux de dents.
